# EUROPEAN PATENT APPLICATION

(11) **EP 1 568 370 A1**
(43) Date of publication of application: **31.08.2005**
(21) Application number: 04030858.7
(22) Date of filing: 28.12.2004
(51) Int. Cl.: A61K 31/7056, A61K 31/327, A61P 17/00, A61K 47/08, A61P 31/04

(54) **Topical antibacterial formulations**

(30) Priority: 27.02.2004 US 787231
(71) Applicant: STIEFEL LABORATORIES, INC., Coral Gables, FL 33134 (US)
(72) Inventor: Popp, Karl F., Schodack Landing New York 12156 (US)
(74) Representative: Welch, Andreas

(57) **Abstract**

Topical composition comprising a storage-stable mixture of a benzoyl peroxide dispersion and clindamycin or pharmaceutically acceptable salts or esters thereof for treating a bacterial disorder other than acne. These methods also contemplate the reduction or elimination of particular bacteria from affected skin areas, thereby reducing skin lesions or infections.

## Description

This application claims priority to U.S. Patent Application Serial No. 10/787,231, filed on February 27, 2004, the contents of which are hereby incorporated by reference in their entirety.

### FIELD OF THE INVENTION

The present subject matter relates to methods of using a topical composition comprising a storage-stable mixture of a benzoyl peroxide dispersion and clindamycin or pharmaceutically acceptable salts or esters thereof for treating a bacterial disorder. These methods also contemplate the reduction or elimination of particular bacteria from affected skin areas, thereby reducing skin lesions or infections.

### BACKGROUND OF THE INVENTION

Human skin is a composite material of the epidermis and the dermis. The topmost part of the epidermis is the stratum corneum. This layer is the stiffest layer of the skin, as well as the one most affected by the surrounding environment. Below the stratum corneum is the internal portion of the epidermis. Below the epidermis, the topmost layer of the dermis is the papillary dermis, which is made of relatively loose connective tissues that define the micro-relief of the skin. The reticular dermis, disposed beneath the papillary dermis, is tight, connective tissue that is spatially organized. The reticular dermis is also associated with coarse wrinkles. At the bottom of the dermis lies the subcutaneous layer.

The principal functions of the skin include protection, excretion, secretion, absorption, thermoregulation, pigmentogenesis, accumulation, sensory perception, and regulation of immunological processes. These functions are detrimentally affected by, for example, dryness, yeast, and structural changes in the skin, such as due to aging and excessive sun exposure.

Within the skin are numerous different indigenous bacteria. Included among these bacteria are various micrococci and diptheroids normally present in healthy skin. However, the skin may also be infected with various other bacteria that convey adverse effects to the skin.

Such bacterial infections can result in one instance from a collection of sebum in follicular openings and follicular canals. Sebum produced from the sebaceous glands can agglomerate and form solid plugs known as comedones in skin follicles. This can, in turn, cause hyperkeratosis of the follicular opening, which can completely close off the follicular canal. When comedones form in the follicular opening, bacteria present therein can feed on the sebum, activating the immune system of the body and causing bacterial infections, as well as other noninfectious dermatological diseases or disorders involving the bacteria. These bacterial disorders can cause skin lesions, inflammation, swelling, redness, pustules, cysts, nodules, papules, and a variety of other adverse skin effects and disorders. Similarly, bacterial infection or colonization may be secondary to another skin disease.

In the past, primary bacterial skin infections typically have been treated with oral/systemic antibiotics, including tetracycline, erythromycin, and minocycline. However, such systemic treatments have tended to clear secondary infections relatively slowly. Additionally, systemically delivered antibiotics can at times cause many undesirable side effects, including abdominal cramps, black tongue, fatigue, depression, nausea, and other various side effects.

Accordingly, non-systemic, topically delivered treatments have been proposed to alleviate some of these difficulties. However, when applied topically, certain antibiotics can lose their effectiveness if used for a prolonged period of time due to resistance developed by the bacteria. Further, dermatological bacterial disorders may at times be contrary to effective treatment with topical pharmaceutical compositions in view of the barrier function of the skin and the difficulty of drug absorption thereby.

Various topical antibiotic compositions used for the potential treatment of bacterial disorders are known in the art. Some of these topical compositions have separately contained the antibiotics tetracycline, erythromycin, and clindamycin, as well as benzoyl peroxide, which exerts its antibacterial action via its potent oxidizing properties. However, the strong oxidizing properties of the peroxide component can result in unstable compositions. Benzoyl peroxide also can act as a sebosuppressant, an irritant, and a comedolytic agent.

One currently available product, Cleocin T® brand clindamycin phosphate topical solution by Pharmacia & Upjohn Company of Kalamazoo, MI, is a topical solution containing 1% of clindamycin phosphate. Cleocin T®, however, has several drawbacks. For one, the formulation contains 50% isopropyl alcohol and water. This formulation often proves to be excessively drying and irritating to the skin. Second, the composition as dispensed by the pharmacist lacks the stability necessary for extended storage at room temperature.

Topical compositions combining at least two active antibacterial agents typically have the further drawback of requiring compounding by the pharmacist and must be refrigerated. After three months of room temperature storage, the compositions lose potency and effectiveness and must be replaced with a new batch.

For example, a currently available combination product is Benzamycin® brand topical gel (Dermik Laboratories, Berwyn, PA) which contains 3% of erythromycin and 5% of benzoyl peroxide. Benzamycin®, however, has several drawbacks. First, the product is supplied to pharmacies as a benzoyl peroxide gel in a first container and erythromycin powder in a second container. The product thus requires compounding by the pharmacist, who must (1) dissolve the erythromycin in alcohol, (2) add the erythromycin solution to the gel, and (3) stir until homogeneous in appearance. Second, the alcohol present in the composition as dispensed in amounts to 16% of the total composition, which has proven to be excessively drying and irritating to the skin, particularly in combination with the benzoyl peroxide. Third, the composition as dispensed by the pharmacist (i.e., after reconstitution or compounding) lacks the stability necessary for extended storage at room temperature. The combination product can be stored under refrigeration for up to three (3) months.

Similarly, the currently available combination product BenzaClin® (Dermik Laboratories, Berwyn, PA) is a topical gel containing 1% of clindamycin and 5% of benzoyl peroxide. Benza-Clin®, however, also has several drawbacks. For example, the product must be compounded by a pharmacist since it is supplied to pharmacies as a benzoyl peroxide gel in a first container and clindamycin powder in a second container. Accordingly, it lacks the stability necessary for extended storage at room temperature since the combined product can only be stored for up to two (2) months. By requiring compounding by pharmacists, it also has variability/impurity problems, which are the result of the drug forming partially dissolved or undissolved aggregates. This causes some patients to report that the product sometimes feels "gritty" when applied to the skin, further exacerbating the inflammation and irritation problem due to skin abrasion. Lastly, this composition must be topically applied at least twice a day to be effective in accordance with label directions.

For these reasons, there remains a need for storage-stable topical compositions that are effective in treating bacterial disorders. In particular, it would be desirable to provide products combining the activity of an antibiotic compound, such as clindamycin, with the activity of benzoyl peroxide, for the treatment of bacterial disorders, with few or none of the disadvantages described above. Such compositions should overcome the formulation and stability problems which have been associated with the prior compositions, and provide improved compositions which are less irritating, easy to formulate, have a smooth consistency after formulation, are substantially uniform, are adequately stable, and have a sufficiently long storage life with or without refrigeration.

### SUMMARY OF THE INVENTION

The present subject matter relates to a method for treating a bacterial disorder in skin of a patient, comprising:
topically administering to the skin of a patient in need thereof a topical composition in an amount effective to treat said bacterial disorder, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said bacterial disorder.

In a preferred embodiment, the present subject matter also relates to a method for reducing or eliminating bacteria from skin of a patient, comprising:
topically administering to skin of a patient infected with said bacteria a topical composition in an amount effective to reduce or eliminate said bacteria, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in reducing or eliminating said bacteria.

In another preferred embodiment, the present subject matter relates to a method for treating a bacterial disorder in a patient having sensitive skin, comprising:
topically administering to sensitive skin areas of a patient in need thereof a topical composition in an amount effective to treat said bacterial disorder, wherein said composition comprises:
   a storage-stable mixture of a benzoyl peroxide dispersion, clindamycin or a pharmaceutically acceptable salt or ester thereof, and a pharmaceutically acceptable carrier;
wherein said topical composition has a viscosity that enhances the effectiveness of the topical composition in treating said bacterial disorder in said sensitive skin.

In a further preferred embodiment, the present subject matter relates to a method for treating a topical bacterial infection in skin of a patient, comprising:
topically administering to the skin of a patient in need thereof a topical composition in an amount effective to treat said topical bacterial infection, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said topical bacterial infection.

In yet another preferred embodiment, the present subject matter relates to a method for treating impetigo in skin of a patient, comprising:
topically administering to the skin of a patient in need thereof a topical composition in an amount effective to treat said impetigo, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said impetigo.

In still another preferred embodiment, the present subject matter relates to a method for treating folliculitis in skin of a patient, comprising:
topically administering to the skin of a patient in need thereof a topical composition in an amount effective to treat said folliculitis, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said folliculitis.

In still yet another preferred embodiment, the present subject matter relates to a method for treating erythrasma in skin of a patient, comprising:
topically administering to the skin of a patient in need thereof a topical composition in an amount effective to treat said erythrasma, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said erythrasma.

In a further preferred embodiment, the present subject matter relates to a method for treating a bacterial disorder in skin of a patient, comprising:
topically administering to the skin of a patient in need thereof a topical composition in an amount effective to treat said bacterial disorder, wherein said topical composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said bacterial disorder;
and wherein said topical composition is administered concomitantly or sequentially with an additional active agent effective to treat said bacterial disorder.

In another preferred embodiment, the present subject matter relates to the use of benzoyl peroxide and clindamycin or a pharmaceutically acceptable salt or ester thereof in the preparation of a medicament in the form of a topical composition for treating a bacterial disorder other than acne in skin of a patient, wherein said topical composition comprises:
an amount effective to treat said bacterial disorder other than acne of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said bacterial disorder other than acne.

In still another preferred embodiment, the present subject matter relates to the use of benzoyl peroxide and clindamycin or a pharmaceutically acceptable salt or ester thereof in the preparation of a medicament in the form of a topical composition for treating a bacterial disorder other than acne in skin of a patient, wherein said topical composition comprises:
an amount effective to treat said bacterial disorder other than acne of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said bacterial disorder other than acne and wherein said topical composition is formulated to be suitable for topical application concomitantly or sequentially with an additional active agent effective to treat said bacterial disorder other than acne.

In yet another preferred embodiment, the present subject matter relates to the use of benzoyl peroxide and clindamycin or a pharmaceutically acceptable salt or ester thereof in the preparation of a medicament in the form of a topical composition for treating a topical bacterial infection other than acne in skin of a patient, wherein said topical composition comprises:
an amount effective to treat said topical bacterial infection other than acne of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said topical bacterial infection other than acne.

In a further preferred embodiment, the present subject matter relates to the use of benzoyl peroxide and clindamycin or a pharmaceutically acceptable salt or ester thereof in the preparation of a medicament in the form of a topical composition for treating impetigo in skin of a patient, wherein said topical composition comprises:
an amount effective to treat said impetigo of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said impetigo.

In still yet another preferred embodiment, the present subject matter relates to the use of benzoyl peroxide and clindamycin or a pharmaceutically acceptable salt or ester thereof in the preparation of a medicament in the form of a topical composition for treating folliculitis in skin of a patient, wherein said topical composition comprises:
an amount effective to treat said folliculitis of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said folliculitis.

In another preferred embodiment, the present subject matter relates to the use of benzoyl peroxide and clindamycin or a pharmaceutically acceptable salt or ester thereof in the preparation of a medicament in the form of a topical composition for treating erythrasma in skin of a patient, wherein said topical composition comprises:
an amount effective to treat said erythrasma of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said erythrasma.

In still another preferred embodiment, the present subject matter relates to the use of benzoyl peroxide and clindamycin or a pharmaceutically acceptable salt or ester thereof in the preparation of a medicament in the form of a topical composition for reducing or eliminating bacteria from skin of a patient other than bacteria causing acne in said skin, wherein said topical composition comprises:
an amount effective to reduce or eliminate said bacteria other than bacteria causing acne in said skin of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in reducing or eliminating said bacteria other than bacteria causing acne in said skin.

In yet another preferred embodiment, the present subject matter relates to the use of benzoyl peroxide and clindamycin or a pharmaceutically acceptable salt or ester thereof in the preparation of a medicament in the form of a topical composition for treating a bacterial disorder other than acne in sensitive skin of a patient, wherein said topical composition comprises:
an amount effective to treat said bacterial disorder other than acne in said sensitive skin of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said bacterial disorder other than acne and wherein said composition is formulated to be suitable for topical application to sensitive skin areas, irritated skin areas, or inflamed skin areas of said patient.

### DETAILED DESCRIPTION OF THE INVENTION

### Definitions

The phrase "bacterial disorder" as used herein refers to any condition, infection, disease, or disorder caused by a bacteria.

The phrase "skin disorder" as used herein refers to any condition, infection, disease, or disorder which afflicts the skin of a patient.

The term "sensitivity" refers to the degree of skin irritation or skin inflammation, as exemplified by parameters in suitable assays for measuring sensitivity, inflammation, irritation, and the like. One such assay is the Jordan-King assay, as set forth in Jordan, W.P. 1994, Jordan/King modification of the Draize Repeat Insult Patch Test, Clairol Study #94046, Test Dates 10/3/94-11/11/94, the entire contents of which are hereby incorporated by reference.

The term "commercial purposes" refers to any purposes requiring any length of time or storage condition in accordance with the U.S. Food and Drug Administration (FDA) rules or regulations, including shipping time, storage, distribution, and refrigeration.

### Compositions

The present subject matter relates to methods of using various topical compositions for treating a bacterial disorder in a patient. These topical compositions preferably comprise a storage-stable mixture of a benzoyl peroxide dispersion, clindamycin or a pharmaceutically acceptable salt or ester thereof, and a pharmaceutically acceptable carrier. Further, the topical composition preferably has a viscosity that enhances the effectiveness of the topical composition in treating the bacterial disorder.

The benzoyl peroxide component of the compositions used herein is introduced as a dispersion. The benzoyl peroxide included in this dispersion is pharmaceutical grade benzoyl peroxide. Further, the benzoyl peroxide in the dispersion may be in the form of a slurry of a finely divided powder, or in the form of a hydrous granular material. Preparation of suitable benzoyl peroxide constituents is well described in the medical and patent literature.

The benzoyl peroxide component of the compositions used herein is generally present in an amount of between about 0.5% to about 20% by weight of the total composition of benzoyl peroxide. In a preferred embodiment, the compositions used herein contain from about 1% to about 12.5% by weight of the total composition of benzoyl peroxide. In a particularly preferred embodiment, the compositions used herein contain about 1.5% to about 6.25% by weight of benzoyl peroxide. The compositions used herein are unobvious in that they can be produced having a standard deviation of benzoyl peroxide present within ± 0.07.

Additionally, the compositions used herein effectively maintain a benzoyl peroxide composition having not more than 0.01% by weight of benzoyl peroxide impurities, excluding solvents.

In a preferred embodiment, the benzoyl peroxide used is about 65% to about 80% pure, the remainder being purified water.

Prior to mixing, the benzoyl peroxide dispersion has a preferred viscosity of about 60,000 to about 250,000 centipoises. In a particularly preferred embodiment, the benzoyl peroxide dispersion has a viscosity of about 110,000 to about 220,000 centipoises prior to mixing.

The clindamycin component of the compositions used in the present methods is preferably a pharmaceutical grade salt or ester of clindamycin. Pharmaceutically acceptable salts, esters, or solvates of clindamycin refer to those which possess the desired pharmacological activity and which are neither biologically nor otherwise undesirable. The salts, esters, or solvates can be formed with inorganic or organic acids such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, gluconate, glycerophosphate, hemisulfate, heptanoate, hexanoate, hydrochloride, hydrobromide, hydroiodide, 2-hydroxyethanesulfonate, lactate, maleate, methanesulfonate, naphthylate, 2-naphthalenesulfonate, nicotinate, oxalate, sulfate, thiocyanate, tosylate and undecanoate.

Base salts, esters, or solvates useful herein include ammonium salts, alkali metal salts such as lithium, sodium and potassium salts, alkaline earth metal salts such as calcium and magnesium salts, salt with organic bases such as dicyclohexylamine salts, N-methyl-D-glucamine, and salts with amino acids such as arginine, lysine, and so forth. Also the basic nitrogen-containing groups can be quarternized with such agents as: 1) lower alkyl halides, such as methyl, ethyl, propyl, and butyl chloride, bromides and iodides; 2) dialkyl sulfates like dimethyl, diethyl, dibutyl and diamyl sulfates; 3) long chain alkyls such as decyl, lauryl, myristyl and stearyl substituted with one or more halide such as chloride, bromide and iodide; and 4) aryl or arylalkyl halides like benzyl and phenethyl bromide and others.

Clindamycin phosphate (ester) and clindamycin hydrochloride (salt) are preferred pharmaceutically acceptable salts and esters of clindamycin which can be used in the present compositions due to their compatibility with gelling agents and extensive history of topical use.

The clindamycin component of the compositions used herein is generally present at an amount of from about 0.90% to about 2.5% by weight of the total composition. In a preferred embodiment, the compositions used herein contain between about 0.5% and about 1.5% by weight clindamycin. In a particularly preferred embodiment, the compositions used herein contain about 1.2% by weight clindamycin. The compositions used in the present methods are unique in that they can be produced having a standard deviation of clindamycin present within ± 0.015.

Additionally, the compositions used herein effectively maintain a clindamycin composition having not more than 0.02% by weight of clindamycin degradates.

In the final composition, the ratio of benzoyl peroxide to clindamycin may be from about 1.8:1 to 12:1. Particularly useful are compositions wherein the ratio of benzoyl peroxide to clindamycin is from about 4:1 to about 5:1. Further, the final compositions preferably have a final viscosity of about 50,000 to about 200,000 centipoises. In a particularly preferred embodiment, the final compositions have a final viscosity of about 100,000 to about 180,000 centipoises.

In a particularly preferred embodiment of the compositions used herein, the final topical composition has a viscosity lower than the viscosity of the benzoyl peroxide dispersion before mixing. This final viscosity that is lower than the viscosity of the benzoyl peroxide dispersion demonstrates that the compositions used in the present methods are easier to mix together, contain less degradates, and have a greater degree of uniformity than those compositions previously known in the art.

In another preferred embodiment, the final compositions exhibit a final pH of about 4.5 to about 5. In a particularly preferred embodiment, the final compositions exhibit a final pH of about 4.6 to about 4.8. This narrowly tailored pH is in part responsible for the advanced storage stability of the present compositions in comparison to those previously known in the art. In view of these particular viscosity and pH features, the compositions used in the present methods are storage-stable for commercial purposes.

In a preferred embodiment, the compositions used in the present methods are particularly stable when stored at a temperature of less than about 30 °C.

The present compositions do not require compounding at the time of dispensing and maintain stability indefinitely depending on the storage temperature, despite the relative incompatibility of benzoyl peroxide and clindamycin. This represents a distinct advantage over the formulations previously known in the art.

The present methods preferably use compositions formulated for either once-per-day or twice-per-day administration. In a preferred embodiment, the once-per-day administration is in the morning or A.M. to increase patient compliance and to account for skin conditions most favorable to reducing inflammation. An additional advantage of administration in the morning is the minimization of the risk of bleaching fabrics occasionally seen when a patient puts a benzoyl peroxide product on their face at night and the medication comes in contact with a "colored" pillow case or sheet, etc. resulting in a white spot on the fabric. The benzoyl peroxide dispersion, as well as the final composition, may take the form of a gel, cream, lotion, suspension, emulsion, ointment, foam, or mixtures thereof. Other cosmetic treatment compositions known to those skilled in the art, including liquids and balms, are additionally contemplated as falling within the scope of the present subject matter. Further, the present subject matter contemplates applying any of these compositions with an applicator. Non-limiting examples of useful applicators include a pledget and a pad. Additionally, the present subject matter further contemplates that any of these topical composition are provided in a package of less than 5 g topical composition as a unit of use.

Emulsions, such as oil-in-water or water-in-oil systems, as well as a base (vehicle or carrier) for the topical formulation is selected to provide effectiveness of the active ingredient and/or avoid allergic and irritating reactions (e.g., contact dermatitis) caused by ingredients of the base or by the active ingredients.

Creams useful in the compositions used herein may also be semisolid emulsions of oil and water. They are easily applied and vanish when rubbed into the skin.

Lotions useful in the compositions used herein include suspensions of powdered material in a water or alcohol base (e.g., calamine), as well as water-based emulsions (e.g., some corticosteroids). Convenient to apply, lotions are also cool and help to dry acute inflammatory and exudative lesions.

Suitable lotions or creams containing the active compound may be suspended or dissolved in, for example, a mixture of one or more of the following: mineral oil, sorbitan monostearate, polysorbate 60 (polyoxyethylene 20 sorbitan monostearate), cetyl ester wax, cetearyl alcohol, 2-octyldodecanol, benzyl alcohol, and water.

Ointments which are useful herein are oleaginous and contain little if any water; feel greasy but are generally well tolerated; and are best used to lubricate, especially if applied over hydrated skin. These ointments are preferred for lesions with thick crusts, lichenification, or heaped-up scales and may be less irritating than cream formulations for some eroded or open lesions (e.g., stasis ulcers). Drugs in ointments are often more potent than in creams.

The compounds can be formulated into suitable ointments containing the compounds suspended or dissolved in, for example, mixtures with one or more of the following: mineral oil, liquid petrolatum, white petrolatum, propylene glycol, polyoxyethylene polyoxypropylene compound, emulsifying wax and water.

In severe cases, occlusive therapy may be useful herein, particularly where the trunk or extremities are affected by the rosacea. Covering the treated area with a nonporous occlusive dressing increases the absorption and effectiveness of topical corticosteroids. Usually, a polyethylene film (plastic household wrap) is applied overnight over cream or ointment, since a cream or ointment is usually less irritating than lotion in occlusive therapy. Plastic tapes may be impregnated with drug and is especially convenient for treating isolated or recalcitrant lesions; children and (less often) adults may experience pituitary and adrenal suppression after prolonged occlusive therapy over large areas.

Suitable gelling agents which may be useful in the present compositions include aqueous gelling agents, such as neutral, anionic, and cationic polymers, and mixtures thereof. Exemplary polymers which may be useful in the instant compositions include carboxy vinyl polymers, such as carboxypolymethylene. A preferred gelling agent is Carbopol® brand polymer such as is available from Noveon Inc., Cleveland, OH. Suitable gelling agents include Carbopol® polymers. Carbopol® polymers are high molecular weight, crosslinked, acrylic acid-based polymers. Carbopol® homopolymers are polymers of acrylic acid crosslinked with allyl sucrose or allylpentaerythritol. Carbopol® copolymers are polymers of acrylic acid, modified by long chain (C10-C30) alkyl acrylates, and crosslinked with allylpentaerythritol.

Other suitable gelling agents include cellulosic polymers, such as gum arabic, gum tragacanth, locust bean gum, guar gum, xanthan gum, cellulose gum, methylcellulose, hydroxyethylcellulose, hydroxypropylcellulose, and hydroxypropylmethylcellulose.

The compositions used herein may further contain at least one additional inactive ingredient in an amount effective to enhance the stability of said compositions. Any non-toxic, inert, and effective carrier may be used to formulate the compositions used herein. Well-known carriers used to formulate other therapeutic compounds for administration to humans will be useful in these compositions. Pharmaceutically acceptable carriers, excipients and diluents in this regard are well known to those of skill in the art, such as those described in The Merck Index, Thirteenth Edition, Budavari et al., Eds., Merck & Co., Inc., Rahway, N.J. (2001); the CTFA (Cosmetic, Toiletry, and Fragrance Association) International Cosmetic Ingredient Dictionary and Handbook, Tenth Edition (2004); and the "Inactive Ingredient Guide", U.S. Food and Drug Administration (FDA) Center for Drug Evaluation and Research (CDER) Office of Management, January 1996, the contents of which are hereby incorporated by reference in their entirety. Examples of such useful pharmaceutically acceptable excipients, carriers and diluents include distilled water, physiological saline, Ringer's solution, dextrose solution, Hank's solution, and DMSO, which are among those preferred for use herein.

These additional components, as well as effective formulations and administration procedures, are also well known in the art and are described in standard textbooks, such as Goodman and Gillman's: The Pharmacological Bases of Therapeutics, 8th Ed., Gilman et al. Eds. Pergamon Press (1990); Remington's Pharmaceutical Sciences, 17th Ed., Mack Publishing Co., Easton, Pa. (1990); and Harry's Cosmeticology, 8th ed. (2000, Chemical Publishing Co., Inc., New York, NY 10016), each of which are incorporated by reference herein in their entirety.

Examples of preferred inactive ingredients that can be used in the present compositions include but are not limited to carbomer, disodium monolauryl sulfosuccinate, disodium ethylenediaminetetraacetic acid (disodium EDTA), methyl paraben, poloxamer, glycerin, dimethicone, hydrated silica, sodium hydroxide, purified water, and mixtures thereof.

Other ingredients which may optionally be provided in the instant topical compositions include humectants, such as propylene glycol; solvents, such as alcohol (both organic and inorganic alcohol); and anti-microbial preservatives, such as methylparaben and propylparaben. The topical compositions may also include an organic or inorganic base, such as sodium hydroxide, which is used to adjust the pH of the initial components and the final product.

### Additional Active Ingredients

The subject matter described herein further contemplates administering an additional active ingredient readily known to those of skill in the art as useful in the topical treatment of skin disorders or conditions. These additional active ingredients are administered topically or orally either concomitantly or sequentially with the above described topical compositions comprising benzoyl peroxide and clindamycin for the treatment of bacterial disorders. Accordingly, the additional active ingredient is administered with the topical composition either in adjunctive or co-therapy. That is, the additional active ingredient can either be administered as a component of the topical composition or as part of a second, separate composition. This second, separate composition can be either an oral or a topical composition.

Exemplary additional active ingredients include, but are not limited to, other macrolide antibiotics, bactericidal drugs, bacteriostatic drugs, cleansing agents, absorbents, anti-infective agents, anti-inflammatory agents, astringents (drying agents that precipitate protein and shrink and contract the skin), emollients (skin softeners), moisturizers, keratolytics (agents that soften, loosen, and facilitate exfoliation of the squamous cells of the epidermis), retinoids, salts thereof, and mixtures thereof.

Exemplary additional macrolide antibiotics contemplated herein include, but are not limited to, azithromycin, clarithromycin, erythromycin, lincomycin, salts thereof, and mixtures thereof. The macrolides are similar in structure and activity. All the macrolides are easily absorbed and all are primarily bacteriostatic by inhibiting bacterial protein synthesis. These drugs are active against aerobic and anaerobic gram-positive cocci, with the exception of enterococci, and against gram-negative anaerobes, and can be useful herein.

Exemplary bactericidal drugs .(i.e., they kill bacteria) contemplated herein include, but are not limited to, penicillins, cephalosporins, vancomycin, aminoglycosides, quinolones, polymyxins, salts thereof, and mixtures thereof.

Exemplary bacteriostatic drugs (i.e., they slow bacterial growth) contemplated herein include, but are not limited to, erythromycin, tetracyclines, chloramphenicol, lincomycin, clarithromycin, azithromycin, sulfonamides, salts thereof, and mixtures thereof. However, it is well know that some bactericidal drugs may be bacteriostatic against certain microorganisms and vice versa. These drugs are well known in the art and may be found, for example, in The Merck Manual of Diagnosis and Therapy, 13^{th} edition, Section 13, Chapter 153 Anti-bacterial Drugs, 2001, incorporated herein by reference in its entirety.

Furthermore, the formulation may be used with adjunct therapies and treatments, such as pre-washing with common soaps, and mild detergents. However, selection is important when treating particular bacterial skin disorders since antibacterial soaps and abrasive soaps may increase irritation of certain bacterial infections and make it difficult to use follicular drugs. Such follicular drugs may include topical antibiotics and antiseptics, as well as intralesional corticosteroids.

Another combination therapy involves 20% azelaic acid in cream form, which has antiproliferative and antibacterial effects.

An additional combination therapy contemplated herein is topical tretinoin (retinoic acid) in 0.025%, 0.05%, or 0.1% cream, 0.05% liquid, or 0.01% or 0.025% gel. Also, a new topical retinoid, Differin® brand adapalene 0.1% gel, Galderma Laboratories, San Antonio, TX, was recently approved in the USA and may be useful since it may be slightly less irritating than topical tretinoin. Other retinoids which may be useful in combination therapy include Panretin®, containing alitretinoin, and Targretin®, containing bexarotene, Ligand Pharmaceuticals Inc., San Diego, CA. Since retinoids must be applied carefully and at night to avoid excessive irritation, a regimen in combination with these drugs may be used over time to achieve results. For example, retinoid therapy may be initiated and then followed on with once a day treatment in accordance with the present methods. Exposure to sunlight when using retinoids and concurrent use of other drugs are restricted to prevent severe irritation. However, a back-to-back alternating regimen over a period of weeks or months time may be useful.

Other topical drugs including OTC drugs, various sulfur-resorcinol combinations, and oral antibiotics may also be helpful in combination with the present compositions when treating bacterial disorders.

Accordingly, a preferred embodiment of the present subject matter additionally relates to a method for the treatment of a bacterial disorder in a patient in need thereof, comprising administering a combination of benzoyl peroxide and clindamycin to said patient, wherein said combination contains a low level of lincomycin phosphate sulfoxide and lincomycin sulfoxide, i.e. components that can be derived from clindamycin, or can be modified to form clindamycin.

### Methods of Treating Bacterial Disorders

The compositions described herein are preferably used in methods of treating a bacterial disorder in a patient. To carry out these methods, the present compositions are preferably topically administered to skin of a patient affected by the bacterial disorder. In preferred aspects, the patient is a human. In most preferred embodiments, the bacterial disorder is a skin or dermatological bacterial disorder, i.e. it affects the skin.

While not being limited to any specific cause of the bacterial disorder, in a preferred embodiment the present methods involve the treatment of bacterial disorders that exhibit effects selected from the group consisting of skin lesions, inflammation, swelling, redness, pustules, cysts, nodules, papules, hypertrophy of the sebaceous glands, combinations thereof, and a variety of other skin effects.

In a particularly preferred embodiment, the bacterial disorder is selected from the group consisting of topical bacterial infections, impetigo, folliculitis, erythrasma, bacterial vaginosis, and combinations thereof. Preferred examples of the topical bacterial infections include those selected from the group consisting of primary infections, secondary infections, and combinations thereof.

In another preferred embodiment, the bacterial disorder is caused by bacteria selected from the group consisting of gram positive bacteria, gram negative bacteria, and a combination thereof. Accordingly, the present methods additionally contemplate reducing or eliminating these bacteria from skin of a patient.

Preferred, non-limiting examples of gram positive bacteria treatable herein include those selected from the group consisting of *Streptococcus* sp., *Micrococcus* sp., *Staphylococcus* sp., *Bacillus* sp., *Corynebacterium* sp., *Clostridium* sp., *Listeria monocytogenes,* and combinations thereof.

Preferred, non-limiting examples of treatable *Streptococcus* sp. contemplated according to the present methods are those selected from the group consisting of *S. viridans, S. agalactiae, S. pyogenes, S. faecalis, S. durans, S. faecium, S. mutans, S. sanguis, S. salivarius, S. mitior, S. constellatus, S. intermedius, S. anginosus, S. milleri, S. iniae, S. pneumoniae,* and combinations thereof.

Preferred, non-limiting examples of treatable *Staphylococcus* sp. contemplated according to the present methods are those selected from the group consisting of *S. aureus, S. epidermis,* and combinations thereof.

Preferred, non-limiting examples of treatable *Corynebacterium* sp. contemplated according to the present methods are those selected from the group consisting of *C*. *minutissimum, C. jeikeium, C. urealyticum, C. xerosis,* and combinations thereof.

Preferred, non-limiting examples of treatable *Clostridium* sp. contemplated according to the present methods are those selected from the group consisting of *C. perfringens, C. tetani, C. botulinum, C. difficile,* and combinations thereof.

Preferred, non-limiting examples of gram negative bacteria treatable herein include those selected from the group consisting of *Proteus* sp., *Escherichia coli, Pseudomonas* sp., *Pasteurella multocida, Aeromonas hydrophila, Vibrio vulnificus,* and combinations thereof.

A preferred, non-limiting example of a treatable *Pseudonomas* sp. contemplated according to the present methods is *P. aeruginosa.*

After elimination of the bacterial disorder, application of the present topical compositions may continue once a day to maintain the skin as free of the initial or other bacterial disorders.

After the present compositions carry out their bactericidal activity on these skin bacteria, inflammatory responses to the bacteria begin to diminish. However, remnants of the dead bacteria may still elicit some skin reaction until the cleanup processes of the body fully remove them, a process that can at times require six to eight weeks. After prolonged intervals of freedom from symptoms of bacterial disorders, should classic signs begin to reappear, treatment can be repeated. Such re-treatments should not be necessary more than one or two times per year.

In a further preferred embodiment, the present methods involve treating bacterial disorders in a patient having sensitive skin. In this regard, the present topical compositions are topically applied to sensitive skin areas, irritated skin areas, or infected skin areas.

In another preferred embodiment, the topical application of the present compositions reduces the redness, flushing, and blushing associated with sensitive skin.

The treatment for bacterial disorders described herein is also capable of treating other skin conditions, infections, diseases, or disorders associated with, related to, or commonly further occurring in skin affected by bacterial disorders. These other skin disorders can include but are not limited to antimicrobial resistant bacterial infections, atopic dermatitis, bromhidrosis, chronic paronychia, desquamative inflammatory vaginitis, Fox Fordyce Disease, Hailey-Hailey Disease, Hidradenitits suppurativa, intertrigo, nummular dermatitis, otopyorrhea, perioral dermatitis, angular chelitis, pre-surgical skin prophylaxis, Pseudofolliculitis barbae, psoriasis, Pyoderma gangrenosum, seborrheic dermatitis, skin ulcers, and combinations thereof. The present methods contemplate treating these additional skin disorders either separately from and/or in combination with the above bacterial disorders.

In a preferred embodiment, these other skin disorders improve following treatment with the present compositions.

In another preferred embodiment, the present subject matter further relates to a method for the treatment of bacterial disorders in a patient in need thereof, comprising administering a combination of benzoyl peroxide and clindamycin which has been refrigerated to said patient. This combination has a specific degradation profile, in accordance with the data submitted below.

### Process for Preparing

The present subject matter further relates to a process for preparing a storage-stable topical composition for treating bacterial disorders, which comprises the steps of:
forming at a temperature of about 15 to about 25 °C a benzoyl peroxide intermediate dispersion having between about 5.9% and about 7.2% benzoyl peroxide and having a viscosity of about 60,000 to about 250,000 centipoises;
forming at a temperature of about 15 to about 25 °C a clindamycin intermediate solution sufficient to yield a composition which contains between about 0.5% and about 1.5% by weight clindamycin active in the final product; and
mixing said benzoyl peroxide intermediate dispersion and said clindamycin intermediate solution under conditions sufficient to yield a benzoyl peroxide and clindamycin mixture having a final pH of between about 4.5 to about 5.0,
wherein said mixture has a viscosity of about 50,000 to about 200,000 centipoises, and wherein said composition comprises sufficient inactive ingredients to provide storage stability and effectiveness for a treatment period.

The mixture made according to this process preferably comprises a benzoyl peroxide gel intermediate mixed with a clindamycin solution intermediate. The benzoyl peroxide gel intermediate preferably contains between about 5.9% and about 7.2% by weight benzoyl peroxide.

Also, the composition is preferably manufactured to have about 1% to about 3% less water by weight as compared to a topical formulation having one of benzoyl peroxide or clindamycin alone, but not both together. Such formulations unexpectedly result in compositions that exhibit less skin sensitivity.

Referring to the formulation process of the present compositions, a gel is initially formed. The gel is composed of a carbomer, disodium monolauryl sulfosuccinate, and disodium EDTA to which methylparaben is added as a preservative. Purified water is used as a diluent.

After the gel is formed, wetting agents and emollients are added. After the pH is adjusted, the active ingredients are added to form the final compound.

As discussed above, the active ingredients can be added to the inert ingredients at the same time or separately.

The resultant combination maintains stability for a minimum of three months at room temperature (e.g. 22°C) and relative, or ambient, humidity.

### Routes of Administration/Dosage

To be effective, the route of administration for the compositions used in the present methods must readily affect the target areas. In particular, bacterial disorders are known to affect the face, chest, upper back, and extremities.

Dosage levels for the antibiotics and the benzoyl peroxide are well known in the art and are selected to maximize the treatment of the above conditions. The specific dose level for any particular patient will vary depending upon a variety of factors, including the activity of the specific compound employed; the age, body weight, general health, sex and diet of the patient; the time of administration; the rate of excretion; drug combination; the severity of the particular disease being treated; and the form of administration. Typically, in vitro dosage-effect results can provide useful guidance on the proper doses for patient administration. Studies in animal models are also helpful. The considerations for determining the proper dose levels are well known in the art and are incorporated herein for the present subject matter.

Pharmacokinetic parameters such as bioavailability, absorption rate constant, apparent volume of distribution, unbound fraction, total clearance, fraction excreted unchanged, first-pass metabolism, elimination rate constant, half-life, and mean residence time are well known in the art.

Lessening exposure by once-daily administration affects multiple pharmacokinetic parameters and provides the initial mechanism for avoiding skin irritation and inflammation and the other toxicity issues discussed herein. Additional formulations may be prepared which factor in the benefit/risk ratio for a clindamycin and benzoyl peroxide composition. The level of toxicity of these compounds is known and reference is made to the package inserts for Cleocin T® and BenzaClin® and the level of adverse events reported from their clinical trials. In particular, BenzaClin® reported having the following events: dry skin (12%), pruritis (2%), peeling (2%), erythema (1%) and sunburn (1%) as compared to vehicle which reported dry skin (6%), pruritis (<1%), peeling (-), erythema (<1%) and sunburn (-), or roughly twice the number of side effects as vehicle.

Since benzoyl peroxide is a keratolytic, i.e. causes softening and swelling of the cells at the surface of the skin so that the outer layer of the skin peels off or can easily be removed, reducing exposure to it reduces irritation. Upon application, the benzoyl peroxide converts to benzoic acid and has anti-bacterial and anti-fungal properties. Additionally, the low pH of the present formulations may have an additive keratolytic effect on the skin as well as on the anti-bacterial properties. Benzoyl peroxide may also acts as a preservative within the formulation. Clindamycin may degrade at pH higher than pH 6, thus requiring the pH to be maintained below this level, as described herein. The present formulations take these and other factors into account and are manufactured to reduce sensitivity, irritation, and/or inflammation.

Single dosage kits and packages containing once per day amount of composition may be prepared. Single dose, unit dose, and once-daily disposable containers of the present compositions are contemplated as within the scope of the present subject matter.

The present compositions may be formulated for storage in a substantially non-reactive laminated package to enhance stability of the package. This new method of storage provides enhanced package stability in comparison with the previous paper-based packages.

The amount of composition per single packet may range be from about 0.1 mL to about 20.0 mL, preferably between about 0.5 and about 5.0 mL, more preferably between about 1 and about 3 mL.

In particular, the ability to formulate compositions capable of long term storage, without pre-mixing or compounding requirements prior to application, are also contemplated. Specifically, the present compositions remain unexpectedly stable in storage for periods including between about 3 and about 18 months, preferably between about 3 and about 15 months, more preferably between about 3 and about 12 months, and alternately any time period between about 6 and about 18 months. In this regard, while the product may be refrigerated during the distribution and pharmacy storage phases, the product does not need refrigeration for the about 3 months and longer as stated above when stored by the patient at room temperature.

Once-daily disposable packaging may also improve patient compliance, especially for teenagers.

The stability and effectiveness of the topical preparations may last for at least 3 to 18 months at ambient or room temperature. It has been found that the greater the amount of clindamycin in the final product, the greater the stability is maintained. Stability is maintained indefinitely under refrigeration because degradation is slowed through the storage temperature. This improved stability provides pharmacists and other dispensers of medication with a product which no longer requires compounding at the time of dispensing. Because compounding is no longer required, homogeneity is controlled at the point of manufacture, which improves dosing and ultimately compliance. Furthermore, the present compositions do not employ alcohol as a diluent, which eliminates the drying or irritating effects commonly associated therewith.

Stability of the composition is maintained for longer periods of time depending on the amount of clindamycin employed in the final product and the ratio of benzoyl peroxide to clindamycin. For example, when 1.2% of clindamycin is present in the composition, the shelf life can reach from seven to fourteen months at room temperature while maintaining effectiveness. In contrast, when only 1.02% of clindamycin is employed, the shelf life of the product is closer to three months.

Differences in packaging components and manufacturing techniques yield varied formula responses over a period ranging between seven and fourteen months in stability testing as evidenced by the following data:

| Ref No. | BPO/Clindamycin Ratio | Minimum Projected Stability |
|---|---|---|
| A | 5/1.2 | 14 months |
| B | 5/1.2 | 9 months |
| C | 5/1.2 | 7 months |
| D | 5.9/1 | 7 months |
| E | 5/1.02 | 3 months |
| F | 5/1.02 | 3 months |
| BPO = benzoyl peroxide | | |

In addition to the amount of clindamycin as a control over degradation, the temperature at which the composition is stored determines the length of time that the composition remains stable. When the composition is stored at a temperature below ambient temperature (25° C), the stability is maintained indefinitely. For example, storing the compound at 6° C with the proper amount of excess of clindamycin results in an anticipated shelf life of 3 to 5 years.

Advantageously, the final product requires no compounding by the pharmacist. In addition, compliance with exact amounts is possible with a lessened chance of impurities entering the product and contaminating it.

By maintaining the compositions at the present specific pH, the tendency of benzoyl peroxide to oxidize and degrade clindamycin is largely overcome and the product remains stable during storage at room temperature for extended periods, typically several months or longer. Additionally, the present compositions have been found to remain substantially odor free even after storage at room temperature for extended periods. This is surprising since clindamycin solutions frequently develop a strong offensive odor upon aging. The presence of such an odor is unacceptable in topical formulations which are to be applied to a patient's face.

The following examples are illustrative of preferred embodiments and are not to be construed as limiting the subject matter expressed herein. All polymer molecular weights are mean average molecular weights. All percentages are based on the percent by weight of the final delivery system or formulation prepared unless otherwise indicated and all totals equal 100% by weight.

In the examples, the following ingredients are used: carbomers and polymeric emulsifiers (polyacrylates), such as for example Carbopol® 940 from Noveon Inc., Cleveland, OH; disodium monolauryl sulfosuccinate, such as Monamate™ LA-100, Uniqema, New Castle, DE; emulsifier-solubilizer-stabilizers block PEG/PPG co-polymers such as poloxamer 182, also known as Pluracare® L-62, BASF Corporation, Parsippany, NJ; surfactant-emollient-lubricant-plasticizers such as dimethicone also known as Dow Fluid 200®, Dow Corning Corporation, Midland, MI; sequestering agents such as disodium EDTA; and hydrated silica and absorbants.

### EXAMPLE 1

A highly stable gel composition is prepared using the following components. The active ingredients are benzoyl peroxide and clindamycin phosphate. The formulation is prepared to contain 5% by weight benzoyl peroxide and 1.2% by weight clindamycin phosphate as a gel. The remaining components are inert or auxiliary.

| Intermediate Gel Preparation | |
|---|---|
| Ingredient | Parts by Weight |
| Gel: | |
| Purified Water | 86.50% |
| Carbomer | 2.00% |
| Disodium monolauryl sulfosuccinate | 0.04% |
| Disodium EDTA | 0.10% |
| Methylparaben | 0.30% |
| Total: | 88.94% |

The gel is combined with the following to produce the instant composition:

| Formulation to be Administered | |
|---|---|
| Wetting Agents and Emollients: | |
| Poloxamer 182 | 0.20% |
| Glycerin | 4.00% |
| Dimethicone | 0.10% |
| Hydrated Silica | 0.25% |
| Total: | 4.55% |
| | |

| pH Adjustment: | |
|---|---|
| Sodium Hydroxide | 0.31% |
| Total: | 0.31% |
| | |

| Active Ingredients: | |
|---|---|
| Benzoyl Peroxide | 5.00% |
| Clindamycin Phosphate | 1.20% |
| Total: | 6.20% |
| | |
| Total for Composition: | 100.00% |

### EXAMPLE 2

The following composition is obtained when the following component formulations are mixed in equal parts, and later combined to yield the highly stable product.

| Intermediate Gel Preparation | |
|---|---|
| Ingredient | Parts by Weight |
| Gel: | |
| Purified Water | 82.70% |
| Carbomer | 2.00% |
| Disodium monolauryl sulfosuccinate | 0.04% |
| Disodium EDTA | 0.10% |
| Methylparaben | 0.12% |
| Total: | 85.14% |

The gel is combined with the following to produce the instant composition:

| Benzoyl Peroxide Formulation | |
|---|---|
| Wetting Agents and Emollients: | |
| Poloxamer 182 | 0.20% |
| Glycerin | 4.00% |
| Dimethicone | 0.10% |
| Hydrated silica | 0.25% |
| Total: | 4.55% |
| | |

| pH Adjustment: | |
|---|---|
| Sodium Hydroxide | 0.31% |
| Total: | 0.31% |
| | |

| Active Ingredients: | |
|---|---|
| Benzoyl Peroxide | 10.00% |
| Clindamycin Phosphate | --- |
| Total: | 10.00% |
| | |
| Total for Composition: | 100.00% |

| Clindamycin Formulation | |
|---|---|
| Ingredient | Parts by Weight |
| Gel: | |
| Purified Water | 90.30% |
| Carbomer | 2.00% |
| Disodium monolauryl sulfosuccinate | 0.04% |
| Disodium EDTA | 0.10% |
| Methylparaben | 0.30% |
| Total: | 92.74% |

The gel is combined with the following to produce the instant composition:

| Formulation to be Administered | |
|---|---|
| Wetting Agents and Emollients: | |
| Poloxamer 182 | 0.20% |
| Glycerin | 4.00% |
| Dimethicone | 0.10% |
| Hydrated silica | 0.25% |
| Total: | 4.55% |
| | |

| pH Adjustment: | |
|---|---|
| Sodium Hydroxide | 0.31% |
| Total: | 0.31% |
| | |

| Active Ingredients: | |
|---|---|
| Benzoyl Peroxide | --- |
| Clindamycin Phosphate | 2.40% |
| Total: | 2.40% |
| | |
| Total for Composition: | 100.00% |

The resultant mixture is essentially 10% of benzoyl peroxide with essentially 2% clindamycin.

### EXAMPLE 3

Tables 1 and 2 show the stability of the active ingredients. A fourteen-month analysis was performed on a 5.9% benzoyl peroxide and 1% clindamycin gel formulation. Measurements were taken at the end of 3 months and every month thereafter until the 8th month. No measurements were taken at 8 months. Thereafter, measurements were taken at 9, 12, and 14 months. The composition was stored at 3 different temperatures, i.e., 6° C, 25° C, and 30° C. The level of clindamycin was measured at each temperature, as well as the amount of benzoyl peroxide. The results are as follows:

**TABLE 1 -**

| Clindamycin Stability Benzoyl Peroxide 5% (5.9% in formula) and clindamycin 1% (1% in formula) | | | |
|---|---|---|---|
| Clindamycin Concentration (as % w/w) | | | |
| | 6° C | 25° C | 30° C |
| Initial | | 1.01 | |
| 3 months | 0.95 | 0.90 | 0.77 |
| 4 months | 1.01 | 0.95 | 0.79 |
| 5 months | 1.04 | 0.95 | 0.79 |
| 6 months | 0.96 | 0.91 | 0.71 |
| 7 months | 1.05 | 0.92 | 0.70 |
| 9 months | 1.03 | ND | ND |
| 12 months | 0.98 | 0.79 | 0.37 |
| 14 months | 0.98 | 0.76 | 0.27 |
| ND = No Data | | | |

**TABLE 2 -**

| Benzoyl Peroxide Stability Benzoyl Peroxide 5% (5.9% in formula) and clindamycin 1% (1% in formula) | | | |
|---|---|---|---|
| Benzoyl Peroxide (BPO) Concentration (as % w/w) | | | |
| | 6° C | 25° C | 30° C |
| Initial | | 6.13 | |
| 3 months | 5.97 | 5.90 | 5.98 |
| 4 months | 6.07 | 6.05 | 5.98 |
| 5 months | 6.08 | 5.96 | 5.84 |
| 6 months | 6.13 | 6.04 | 5.91 |
| 7 months | 6.23 | 6.19 | 6.06 |
| 9 months | 6.02 | 5.95 | |
| 12 months | 5.95 | 5.89 | 5.63 |
| 14 months | 6.10 | 6.10 | 5.77 |

### EXAMPLE 4

Tables 3 and 4 show the stability of the active ingredients in the composition containing 5% of benzoyl peroxide and 1.2% of clindamycin.

A six-month analysis of the composition was undertaken following the procedure of Example 3 and utilizing a different amount of clindamycin and benzoyl peroxide.

**TABLE 3 -**

| Clindamycin Stability Benzoyl Peroxide 5% (BPO) (5.9% in formula) and clindamycin 1% (1.2% in formula) | | | |
|---|---|---|---|
| Clindamycin Concentration (as % w/w) | | | |
| | 6° C | 25° C | 30° C |
| Initial | | 1.24 | |
| 1 months | 1.25 | 1.24 | 1.15 |
| 2 months | 1.28 | 1.21 | 1.01 |
| 3 months | 1.23 | 1.13 | 0.94 |
| 6 months | 1.21 | 1.05 | ND |

**TABLE 4 -**

| Benzoyl Peroxide Stability Benzoyl Peroxide 5% (BPO) (5.9% in formula) and clindamycin 1% (1.2% in formula) | | | |
|---|---|---|---|
| Benzoyl Peroxide (BPO) Concentration (as % w/w) | | | |
| | 6° C | 25° C | 30° C |
| Initial | | 5.09 | |
| 1 months | 5.10 | 5.02 | 5.08 |
| 2 months | 5.25 | 5.20 | 5.13 |
| 3 months | 5.16 | 5.18 | 4.82 |
| 6 months | 5.07 | 5.06 | ND |
| ND = No Data | | | |

### EXAMPLE 5

Tables 5 through 13 show the stability of the active ingredients. An analysis of at least 24 months was performed following the procedure of Example 3. Measurements were taken after storage for a specified number of months at 6° C followed by storage for 91 days thereafter at 25° C.

The columns in the following tables represent the following components (as % w/w):
Column A - Clindamycin
Column B - Clindamycin HCl
Column C - Clindamycin B-2 Phosphate
Column D - Clindamycin Phosphate Sulfoxide Isomer 1 (Clindamycin Degradate 1)
Column E - Clindamycin Phosphate Sulfoxide Isomer 2 (Clindamycin Degradate 2)
Column F - Lincomycin Phosphate Sulfoxide (Clindamycin Degradate 3)

**TABLE 5 -**

| Benzoyl Peroxide/Clindamycin Stability Benzoyl Peroxide 5% (BPO) and clindamycin 1% | | | | | | | | |
|---|---|---|---|---|---|---|---|---|
| Trial 1 | | | | | | | | |
| | A | B | C | D | E | F | pH range | Package Size |
| Initial | 1.01 | <0.00 5 | 0.012 | 0.03 7 | 0.04 7 | ND | 4.6-4.7 | 5 g |
| 25 months | 0.95 | <0.00 5 | 0.008 | 0.04 1 | 0.07 4 | 0.00 7 | 4.6-4.7 | 5 g |
| 30 months | 0.93 | <0.00 5 | 0.007 | 0.04 4 | 0.08 2 | 0.00 3 | 4.6-4.7 | 5 g |
| 36 months | 0.93 | <0.00 5 | <0.00 1 | 0.05 0 | 0.08 6 | 0.00 3 | 4.6-4.7 | 5 g |
| 48 months | 0.89 | <0.00 5 | <0.00 1 | 0.06 2 | 0.11 1 | 0.00 3 | 4.6-4.8 | 5 g |

**TABLE 6 -**

| Benzoyl Peroxide/Clindamycin Stability Benzoyl Peroxide 5% (BPO) and clindamycin 1% | | | | | | | | |
|---|---|---|---|---|---|---|---|---|
| Trial 2 | | | | | | | | |
| | A | B | C | D | E | F | pH Range | Package Size |
| Initial | 1.02 | <.0.005 | 0.013 | 0.01 1 | 0.016 | ND | 4.6-4.7 | 45 g |
| 21 months | 0.96 | <0.005 | <0.00 1 | 0.04 6 | 0.070 | 0.008 | 4.6-4.8 | 45 g |
| 24 months | 0.95 | <0.005 | 0.008 | 0.04 8 | 0.084 | 0.008 | 4.6-4.7 | 45 g |
| 30 months | 0.93 | <0.005 | 0.007 | 0.04 5 | 0.083 | 0.003 | 4.6-4.7 | 45 g |
| 36 months | 0.93 | <0.005 | <0.00 1 | 0.04 8 | 0.086 | 0.003 | 4.6-4.8 | 45 g |

**TABLE 7 -**

| Benzoyl Peroxide/Clindamycin Stability Benzoyl Peroxide 5% (BPO) and clindamycin 1% | | | | | | | | |
|---|---|---|---|---|---|---|---|---|
| Trial 3 | | | | | | | | |
| | A | B | C | D | E | F | pH Range | Package Size |
| Initial | 1.05 | <0.005 | 0.008 | <0.001 | 0.005 | ND | 4.8 | 45 g |
| 24 months | 0.92 | <0.005 | <0.00 1 | 0.044 | 0.079 | 0.00 3 | 4.6-4.8 | 45 g |
| 30 months | 0.93 | <0.00 5 | <0.00 1 | 0.048 | 0.087 | 0.00 2 | 4.7-4.8 | 45 g |
| 37 months | 0.91 | <0.00 5 | <0.00 1 | 0.055 | 0.095 | 0.00 3 | 4.5-4.9 | 45 g |
| 42 months | 0.89 | <0.00 5 | <0.00 1 | 0.058 | 0.106 | 0.00 2 | 4.7-4.8 | 45 g |
| 48 months | 0.89 | <0.005 | <0.00 1 | 0.058 | 0.110 | 0.00 3 | 4.6-4.7 | 45 g |

**TABLE 8 -**

| Benzoyl Peroxide/Clindamycin Stability Benzoyl Peroxide 5% (BPO) and clindamycin 1% | | | | | | | | |
|---|---|---|---|---|---|---|---|---|
| Trial 4 | | | | | | | | |
| | A | B | C | D | E | F | pH Range | Package Size |
| 24 months | 0.92 | <0.00 5 | <0.00 1 | 0.04 4 | 0.08 0 | 0.00 4 | 4.7-4.8 | 45 g |
| 30 months | 0.91 | <0.00 5 | <0.00 1 | 0.04 4 | 0.07 5 | 0.00 2 | 4.7-4.8 | 45 g |
| 37 months | 0.90 | <0.00 5 | <0.00 1 | 0.04 9 | 0.09 0 | 0.00 2 | 4.7-4.8 | 45 g |
| 42 months | 0.89 | <0.00 5 | <0.00 1 | 0.05 8 | 0.10 3 | 0.00 2 | 4.7-4.8 | 45 g |

**TABLE 9 -**

| Benzoyl Peroxide/Clindamycin Stability Benzoyl Peroxide 5% (BPO) and clindamycin 1% | | | | | | | | |
|---|---|---|---|---|---|---|---|---|
| Trial 5 | | | | | | | | |
| | A | B | C | D | E | F | pH Range | Package Size |
| Initial | 1.0 1 | <0.00 5 | 0.008 | <0.00 1 | 0.00 5 | ND | 4.7-4.9 | 5 g |
| 24 months | 0.9 1 | <0.00 5 | <0.00 1 | 0.044 | 0.07 8 | 0.00 4 | 4.6-4.8 | 5 g |
| 30 months | 0.9 0 | <0.00 5 | <0.00 1 | 0.040 | 0.07 3 | 0.00 2 | 4.7-4.8 | 5 g |
| 37 months | 0.8 8 | <0.00 5 | <0.00 1 | 0.052 | 0.09 3 | 0.00 3 | 4.4-4.8 | 5 g |
| 42 months | 0.8 9 | <0.00 5 | <0.00 1 | 0.057 | 0.10 4 | 0.00 2 | 4.7-4.8 | 5 g |

**TABLE 10 -**

| Benzoyl Peroxide/Clindamycin Stability Benzoyl Peroxide 5% (BPO) and clindamycin 1% | | | | | | | | |
|---|---|---|---|---|---|---|---|---|
| Trial 6 | | | | | | | | |
| | A B C | B | C | D | E | F | pH Range | Package Size |
| 24 months | 0.9 1 | <0.00 5 | <0.00 1 | 0.04 6 | 0.075 | 0.00 1 | 4.7-4.8 | 45 g |
| 31 months | 0.9 0 | <0.00 5 | <0.00 1 | 0.04 2 | 0.080 | 0.00 1 | 4.7-4.8 | 45 g |
| 36 months | 0.8 8 | <0.00 5 | <0.00 1 | 0.05 5 | 0.101 | 0.00 1 | 4.7-4.8 | 45 g |

**TABLE 11 -**

| Benzoyl Peroxide/Clindamycin Stability Benzoyl Peroxide 5% (BPO) and clindamycin 1% | | | | | | | | |
|---|---|---|---|---|---|---|---|---|
| Trial 7 | | | | | | | | |
| | A | B | C | D | E | F | pH Range | Package Size |
| Initial | 1.015 | <0.005 | <0.00 1 | 0.005 | 0.008 | 0.005 | 4.7 | 45 g |
| 24 months | 0.91 | <0.005 | <0.00 1 | 0.045 | 0.079 | <0.00 1 | 4.764.8 | 45 g |

**TABLE 12 -**

| Benzoyl Peroxide/Clindamycin Stability Benzoyl Peroxide 5% (BPO) and clindamycin 1% | | | | | | | | |
|---|---|---|---|---|---|---|---|---|
| Trial 8 | | | | | | | | |
| | A | B | C | D | E | F | pH Range | Package Size |
| Initial | 1.03 | <0.005 | <0.001 | 0.00 6 | 0.010 | 0.002 | 4.7 | 45 g |

**TABLE 13 -**

| Benzoyl Peroxide/Clindamycin Stability Benzoyl Peroxide 5% (BPO) and clindamycin 1% | | | | | | | | |
|---|---|---|---|---|---|---|---|---|
| Trial 9 | | | | | | | | |
| | A | B | C | D | E | F | pH Range | Package Size |
| Initial | 1.0 4 | <0.005 | <0.001 | 0.006 | 0.009 | 0.002 | 4.7-4.8 | 45 g |

### EXAMPLE 6

A patient suffering from a topical bacterial infection is treated with a topical composition comprising a benzoyl peroxide dispersion and a clindamycin as herein described. It would be expected that the patient would improve his/her condition or recover.

### EXAMPLE 7

A patient suffering from impetigo is treated with a topical composition comprising a benzoyl peroxide dispersion and a clindamycin as herein described. It would be expected that the patient would improve his/her condition or recover.

### EXAMPLE 8

A patient suffering from folliculitis is treated with a topical composition comprising a benzoyl peroxide dispersion and a clindamycin as herein described. It would be expected that the patient would improve his/her condition or recover.

### EXAMPLE 9

A patient suffering from erythrasma is treated with a topical composition comprising a benzoyl peroxide dispersion and a clindamycin as herein described. It would be expected that the patient would improve his/her condition or recover.

The present subject matter being thus described, it will be obvious that the same may be modified or varied in many ways. Such modifications and variations are not to be regarded as a departure from the spirit and scope of the present subject matter and all such modifications and variations are intended to be included within the scope of the following claims.

## Claims

1. Use of benzoyl peroxide and clindamycin or a pharmaceutically acceptable salt or ester thereof in the preparation of a medicament in the form of a topical composition for treating a bacterial disorder or a topical bacterial infection other than acne in skin of a patient, wherein said topical composition comprises:
an amount effective to treat said bacterial disorder other than acne of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating said bacterial disorder other than acne.

2. The use of claim 1, wherein said topical composition is formulated to be suitable for topical application concomitantly or sequentially with an additional active agent effective to treat said bacterial disorder other than acne, preferably either in adjunctive or co-therapy.

3. The use of claim 2, wherein said additional active agent is selected from the group consisting of other macrolide antibiotics, bactericidal drugs, bacteriostatic drugs, cleansing agents, absorbents, anti-infective agents, anti-inflammatory agents, astringents, emollients, moisturizers, keratolytics, retinoids, salts thereof, and mixtures thereof.

4. The use of any of claims 1-3, wherein said bacterial disorder is caused by bacteria selected from the group consisting of gram positive bacteria, gram negative bacteria, and a combination thereof.

5. The use of claim 4, wherein said bacteria is selected from *Streptococcus* sp., *S. viridans, S. agalactiae, S. pyogenes, S. faecalis, S. durans, S. faecium, S. mutans, S. sanguis, S. salivarius, S. mitior, S. constellatus, S. intermedius, S. anginosus, S. milleri, S. iniae, S. pneumoniae, Micrococcus* sp., *Staphylococcus* sp., *S. aureus, S. epidermis, Bacillus* sp., *Corynebacterium* sp., *C. minutissimum, C*. *jeikeium, C. urealyticum, C. xerosis, Clostridium* sp., *C*. *perfringens, C. tetani, C. botulinum, C. difficile, Listeria monocytogenes, Proteus* sp., *Escherichia coli, Pseudomonas* sp., *P*. *aeruginosa, Pasteurella multocida, Aeromonas hydrophila, Vibrio vulnificus,* and combinations thereof.

6. The use of any of claims 1-5, wherein said bacterial disorder or bacterial infection is selected from topical bacterial infections, impetigo, folliculitis, erythrasma, primary infections, secondary infections, and combinations thereof.

7. The use of any of claims 1-6, wherein said composition reduces or eliminates bacteria from skin of a patient other than bacteria causing acne in said skin.

8. The use of any of claims 1-7, wherein said topical composition is formulated to be suitable for topical application to sensitive skin areas, irritated skin areas, or inflamed skin areas of said patient.

9. The use of any of claims 1-8, wherein said topical composition has a viscosity lower than the viscosity of the benzoyl peroxide dispersion before mixing.

10. The use of any of claims 1-9, wherein said topical composition, after addition of the benzoyl peroxide dispersion, has a final viscosity of about 50,000 to about 200,000, preferably about 100,000 to about 180,000, centipoises.

11. The use of any of claims 1-10, wherein said benzoyl peroxide dispersion, prior to making said topical composition, has a viscosity of about 60,000 to about 250,000, preferably about 110,000 to about 220,000, centipoises.

12. The use of any of claims 1-11, wherein said topical composition has a final pH of about 4.5 to about 5, preferably about 4.6 to about 4.8.

13. The use of any of claims 1-12, wherein said topical composition is formulated for once-per-day or twice-per-day administration.

14. The use of any of claims 1-13, wherein said topical composition is selected from the group consisting of a gel, cream, lotion, suspension, emulsion, ointment, foam, and mixtures thereof.

15. The use of claim 14, wherein said topical composition is suitable for application with an applicator.

16. The use of any of claims 1-15, wherein said topical composition is provided in a package of less than 5 g topical composition as a unit of use.

17. The use of any of claims 1-16, wherein said topical composition is stored at a temperature of less than about 30 °C.

18. The use of any of claims 1-17, wherein said benzoyl peroxide is about 65% to about 80% pure.

19. The use of any of claims 1-18, wherein said mixture comprises about 0.5% to about 20% by weight, preferably about 1% to about 12.5% by weight, more preferably about 1.5% to about 6.25% by weight, benzoyl peroxide.

20. The use of any of claims 1-19, wherein said composition further contains at least one additional inactive ingredient in an amount effective to enhance the stability of said composition.

21. The use of claim 20, wherein said at least one additional inactive ingredient is selected from carbomer, disodium monolauryl sulfosuccinate, disodium ethylenediaminetetraacetic acid (disodium EDTA), methyl paraben, poloxamer, glycerin, dimethicone, hydrated silica, sodium hydroxide, purified water, derivatives thereof, and mixtures thereof.

22. The use of any of claims 1-21, wherein said composition is capable of treating a related skin disorder selected from antimicrobial resistant bacterial infections, atopic dermatitis, bromhidrosis, chronic paronychia, Fox Fordyce Disease, Hailey-Hailey Disease, Hidradenitits suppurativa, intertrigo, nummular dermatitis, otopyorrhea, perioral dermatitis, angular chelitis, pre-surgical skin prophylaxis, Pseudofolliculitis barbae, psoriasis, Pyoderma gangrenosum, seborrheic dermatitis, skin ulcers, and combinations thereof.
